# EUROPÄISCHE PATENTANMELDUNG

(11) **EP 1 798 290 A1**
(43) Veröffentlichungstag der Anmeldung: **20.06.2007**
(21) Anmeldenummer: 07104093.5
(22) Anmeldetag: 19.03.2003
(51) Int. Cl.: C12Q 1/02, C12Q 1/26, C12Q 1/527, C12Q 1/48, A61P 43/00

(54) **Tryptophanaminotransferase, Indol-3-pyruvatdecarboxylase und Indol-3-acetaldehydoxidase als neue Targets für Herbizide**

(30) Priorität: 25.03.2002 DE 10213332
(62) Teilanmeldung aus: 03709799.5
(71) Anmelder: BASF Aktiengesellschaft, 67056 Ludwigshafen (DE)
(72) Erfinder: Großmann, Klaus, 67141 Neuhofen (DE); Schiffer, Helmut, 67483 Großfischlingen (DE); Witschel, Matthias, 67098 Bad Dürkheim (DE); Zagar, Cyrill, Kowloon, Hong Kong (CN); Rentzea, Costin, 69123 Heidelberg (DE); Menges, Markus, 34132 Kassel (DE)

(57) **Zusammenfassung**

Gegenstand der vorliegenden Erfindung sind Tryptophanaminotransferase, Indol-3-pyruvatdecarboxylase und Indol-3-acetaldehydoxidase als neue Targets für Herbizide, Testverfahren zur Identifizierung herbizid wirkender Inhibitoren von einem oder mehreren der vorstehend genannten Enzyme, die über dieses Verfahren identifizierten Inhibitoren mit herbizider Wirkung, sowie Verfahren zur Bekämpfung unerwünschten Pflanzenwuchses auf Basis der erfindungsgemäßen Inhibitoren.

## Beschreibung

Gegenstand der vorliegenden Erfindung sind Tryptophanaminotransferase, Indol-3-pyruvatdecarboxylase und Indol-3-acetaldehydoxidase als neue Targets für Herbizide, Testverfahren zur Identifizierung herbizid wirkender Inhibitoren von einem oder mehreren der vorstehend genannten Enzyme, die über dieses Verfahren identifizierten Inhibitoren mit herbizider Wirkung, sowie Verfahren zur Bekämpfung unerwünschten Pflanzenwuchses auf Basis der erfindungsgemäßen Inhibitoren.

Zum Auffinden neuer Herbizide werden die potentiellen Wirkstoffe nach konventioneller Vorgehensweise auf geeignete Testpflanzen appliziert. Diese Methode weist den Nachteil auf, dass zum Testen relativ große Substanzmengen erforderlich sind. Zum anderen werden bei der direkten Applikation auf die zu testenden Pflanzen bereits im ersten Screeningschritt äußerst hohe Anforderungen an die Testsubstanz gestellt, da nicht nur die Inhibierung oder sonstige Modulation der Aktivität eines zellulären Targets(in der Regel ein Protein oder Enzym als Wirkort für ein Herbizid) erforderlich sind, sondern die Substanz dieses Target zunächst überhaupt erreichen muss. Bereits in diesem ersten Schritt muß die Testsubstanz in Bezug auf Aufnahme durch die Pflanze, Permeabilität durch die verschiedenen Zellwände und Membranen, Persistenz zur Erreichung des gewünschten Effektes hohe Anforderungen erfüllen, ehe es schließlich Inhibierung/ Veränderung der Aktivität des gewünschten Zielenzyms kommen kann.

Es ist angesichts dieser Erfordernisse daher nicht überraschend, dass zum einen die Identifizierung neuer Wirkstoffe immer höhere Kosten verursacht, zum anderen immer weniger Wirkstoffe entdeckt werden.

Es war deshalb Aufgabe in der vorliegenden Erfindung, neue Targets für Herbizide zur Verfügung zu stellen.

Überraschender Weise wurde gefunden, dass die Synthese von Indol-3-essigsäure aus L-Tryptophan in Gegenwart von durch die Verbindung der Formel I in vitro gehemmt wird (s. Beispiel 3 und Beispiel 4).

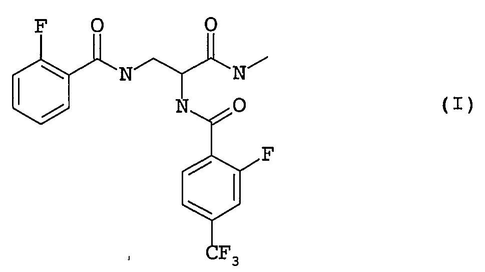

Da Verbindungen der Formel I wirken herbizid wirken (s. Beispiel 4), sind die Tryptophan-Aminotransferase, Indol-3-pyruvat Decarboxylase und die Indol-3-acetaldehydoxidase als Targets für Herbizide geeignet.

Diese Aufgabe wurde gelöst durch die Bereitstellung von Tryptophanaminotransferase, Indol-3-pyruvatdecarboxylase und Indol-3-acetaldehydoxidase als neue Targets für Herbizide. In diesem Rahmen wurden weiterhin Verfahren zur Identifizierung von Substanzen mit herbizider Wirkung bereitgestellt, welche auf der Verwendung von einem oder mehreren Enzymen ausgewählt aus der Gruppe bestehend aus den Enzymen Tryptophanaminotransferase, Indol-3-pyruvatdecarboxylase und Indol-3-acetaldehydoxidase basieren.

Die Biosynthese von Indol-3-essigsäure (Auxin), einem wichtigen Pflanzenhormon, geht von L-Tryptophan aus. Die direkte Vorstufe von L-Tryptophan ist Indol. In allen höheren Pflanzenspezies wird der vom L-Tryptophan ausgehende Syntheseweg wie folgt realisiert: L-Tryptophan wird über die Tryptophan-Aminotransferase in Indol-3-pyruvat umgewandelt, welches über die Indol-3-pyruvat Decarboxylase in Indol-3-acetaldeyd umgewandelt wird. Im Anschluß katalysiert die Indol-3-acetaldehydoxidase die Umwandlung des Indol-3-acetaldeyds in Indol-3-essigsäure, die wiederum durch die Indol-3-buttersäuresynthase in Indol-3-buttersäure umgewandelt wird.. Dabei liegen die Enzyme Tryptophan-Aminotransferase, Indol-3-pyruvat Decarboxylase und Indol-3-acetaldehyd Oxidase in der Regel in einem Multienzymkomplex vor. (Bartel, B. (1997), Ann. Rev. Plant Physiol. Plant Mol. Biol. 48, 51 - 66; Müller, A., Weiler, E.W., (2000), Planta 211, 855 - 863).In Abhängigkeit vom untersuchten Gewebe und Spezies sind aber auch andere untergeordnete Biosynthesewege beschrieben (; Normanly, J., Bartel, B. (1999), Current Opinion in Plant Biology 2, 207 - 213).

Zur Herstellung einer 2,ω-Diaminocarbonsäureverbindungen der Formel I wird man in der Regel eine Verbindung der Formel II, 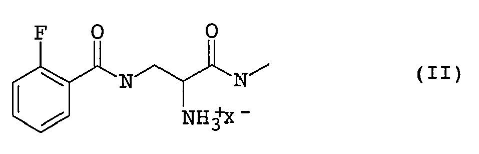 worin Aund X^{⊖} für ein einwertiges Anion oder ein Anionenäquivalent, z.b. Cl^{⊖}, Br^{⊖} oder 1/2 SO₄^{2⊖} einer Mineralsäure, z.b. Cl^{⊖}, Br^{⊖} oder 1/2 SO₄^{2⊖}, steht, mit einem aromatischen Säurehalogenid der Formel III 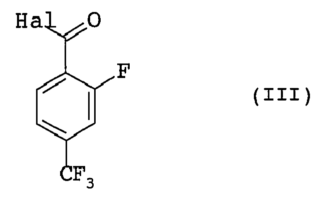 worin Hal für Chlor, Brom oder Iod steht, umsetzen. Die aromatischen Säurehalogenide III sind bekannt und zum Teil kommerziell erhältlich oder können nach bekannten Verfahren hergestellt werden.

Vorzugsweise wird die Umsetzung der Verbindung II mit der Verbindung III in Gegenwart einer Base durchgeführt. Die Base dient zur Neutralisation der bei der Reaktion entstehenden Mineralsäure H-Hal und H-X. Vorzugsweise setzt man die Base in wenigstens äquimolarer Menge, insbesondere in einer Menge von 1 bis 3 Mol pro Mol zu neutralisierender Säure H-Hal und H-X ein.

Vorzugsweise wird die Umsetzung der Verbindung II mit der Verbindung III in einem Lösungs- bzw. Verdünnungsmittel durchgeführt. Hierzu eignen sich Wasser, Diethylether, Tetrahydrofuran, Acetonitril, Essigsäureethylester, Dichlormethan oder Toluol.

Die Reaktionstemperaturen können über einen gewissen Bereich variiert werden, der durch die Stabilität des Säurechlorids III festgelegt wird. Bevorzugt arbeitet man bei Temperaturen im Bereich von 0 bis 30°C.

Die Aufarbeitung erfolgt nach üblichen Methoden z. B. indem man das Reaktionsgemisch mit kaltem Wasser versetzt, die organische Phase abtrennt und nach dem Trocknen unter vermindertem Druck einengt. Der Verbleibende Rückstand kann, falls erforderlich, in üblicher Weise durch Chromatographie oder Kristallisation von eventuell vorhandenen Verunreinigungen befreit werden.

Die Verbindungen der Formel II lassen sich herstellen, indem man zunächst eine teilgeschützte 2,ω-Diaminocarbonsäure der Formel IV oder deren Säureadditionssalz, 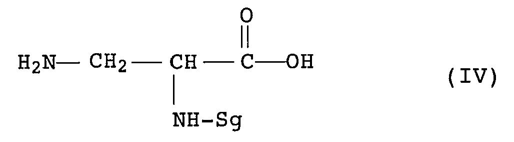 worin Sg für eine Schutzgruppe steht, in einem ersten Schritt mit einem Säurehalogenid der Formel V 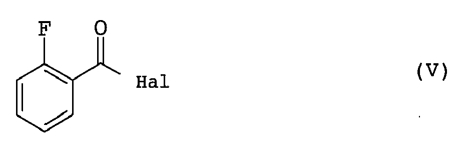 worin Hal für Chlor, Brom oder Iod steht, umsetzt, in einem zweiten Schritt die dabei erhaltene Verbindung der Formel VI 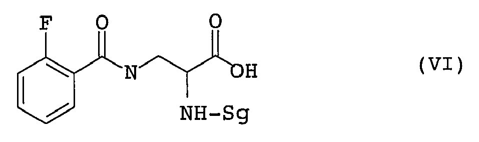 mit einem Amin der Formel CH₃NH₂ in Gegenwart eines geeigneten Kondensationsmittels umsetzt und anschliessend die Schutzgruppe Sg entfernt.

Geeignete Schutzgruppen sind solche, die unter Bedingungen abgespalten werden können, welche nicht zu einer Spaltung der NH-X-Bindung in den Verbindungen der Formel VI führen. Geeignete Schutzgruppen sind aus der Peptidchemie bekannt. Hierzu zählen insbesondere Schutzgruppen, die unter Einwirkung von Säuren, die vorzugsweise eine Säurestärke oberhalb der Essigsäure aufweisen, abgespalten werden, z.B. die tert.-Butyloxycarbonyl-Gruppe, die 1-Adamantyloxycarbonyl-Gruppe und die 2-(Trimethylsilyl)ethoxycarbonyl-Gruppe.

Als Kondensationsmittel für die Umsetzung von Verbindung VI mit dem Amin CH₃NH₂ kommen alle Reagenzien in Frage, die freien Carboxylgruppen aktivieren können wie: Propanphosphonsäure-anhydrid (PPPA, H. Wissmann et al, Angew. Chem. 92, 129 (1980); H. Wissmann, Phosphorus, Sulfur 30, 645 (1986); M. Feigel, J. Am. Chem. Soc 108, 181 (1986)), N-Ethoxycarbonyl-2-ethoxy-1,2-dihydrochinolin (EEDQ, B. Belleau et al, J. Amer. Chem. Soc. 90, 1651 (1968)), Diphenylphosphorylazid (DPPA, Shun-ichi-Yamada et al, J. Am. Chem. Soc. 94, 6203 (1972)) und Diethylphosphorylcyanid (DEPC, Shun-ichi-Ymada et al, Tetrahedron Lett. 18, 1595 (1973)), Carbodiimide (Houben-Weyl, Methoden der Organischen Chemie, Bd. 15/2, Seite 103-115, IV. Auflage, G. Thieme Verlag), um nur einige Kondensationsreagenzien beispielhaft zu nennen. Die dort beschriebenen Reaktionsbedingungen können auf die erfindungsgemässe Umsetzung von Verbindung VI mit dem Amin HNR₂R₃ übertragen werden, so dass auf diese Druckschriften ebenfalls Bezug genommen wird.

Die Entfernung der Schutzgruppe Sg, z.B. der tert.-Butoxycarbonyl-(BOC)-Gruppe, aus den dabei erhaltenen Verbindungen VII 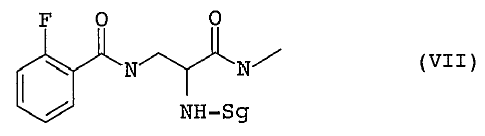 worin Sg die zuvor genannten Bedeutungen aufweist, erfolgt in der Regel mit einer Säure, vorzugsweise mit Hilfe von Trifluoressigsäure z.B. nach den von B. Lundt et al, Int. J. Pept. Protein Res., 12, 258 (1978)) beschriebenen Methoden oder z.B. mit 2N Chlorwasserstoff in Dioxan nach den von R. Andruszkievicz et al, J. Med. Chem. 30, 1715 (1987)) beschriebenen Methoden und liefert in guten Ausbeuten die oben aufgeführten, erfindungsgemäßen Zwischenprodukte der Formel (II).

Die dabei als Ausgansverbindungen eingesetzten Carbonsäure- und Sulfonsäurehalogenide III und V sind bekannt oder lassen sich nach bekannten Methoden herstellen.

2-N-geschützten 2,ω-Diaminosäuren wie Verbindung IV sind ebenfalls bekannt, kommerziell erhältlich oder können nach bekannten Methoden hergestellt werden, z.B. nach N. Kucharczyk et al, Synth. Commun. 19, 1603 (1989); M. Waki et al, Synthesis, 266 (1981) und Lin-Hua Zhang et al, J. Org. Chem. 62, 6918 (1997).

Die Verfahren zur Identifizierung von Substanzen mit herbizider Wirkung umfassen die folgenden Schritte:
a) Inkontaktbringen von einem oder mehreren Enzymen ausgewählt aus der Gruppe bestehend aus den Enzymen Tryptophanaminotransferase, Indol-3-pyruvatdecarboxylase und Indol-3-acetaldehydoxidase mit einer oder mehreren Testsubstanzen unter Bedingungen, die die Bindung der Testsubstanz(en) an eines der vorstehend genannten Enzyme oder an die Nukleinsäuresequenz, die eines vorstehend genannten Enzyme kodiert, erlauben; und
b) Nachweis, ob die Testsubstanzen die Transkription, Translation oder Expression von mindestens einem der vorstehend genannten Enzyme reduzieren oder blockieren; oder
c) Nachweis, ob die Testsubstanzen die Aktivität von mindestens einem der vorstehend genannten Enzyme reduzieren oder blokkieren; oder
d) Nachweis, ob die Testsubstanz an eines der vorstehend genannten Enzyme bindet.

Unter Reduktion oder Blockade der Transkription, Expression, Translation oder der Aktivität in Schritt b) und c) ist eine signifikante Abnahme im Vergleich zur Transkription, Expression, Translation oder der Aktivität bestimmt in Vergleich einem Verfahren, welches sich vom vorstehend genannten Verfahren dadurch unterscheidet, daß man keine Testsubstanz hinzufügt.

Unter einer signifikanten Abnahme ist eine Abnahme von mindestens 10 %, vorteilhaft mindestens 20 %, bevorzugt mindestens 30 %, besonders bevorzugt um mindestens 50 % und ganz besonders bevorzugt um mindestens 70 % bis zu 100 % zu verstehen.

Der Nachweis gemäß Schritt b) des oben stehenden Verfahrens kann anhand von dem Fachmann bekannten Blotting Methoden erfolgen.

Der Nachweis gemäß Schritt d) des oben stehenden Verfahrens kann anhand von Techniken, welche die Wechselwirkung zwischen Protein und Ligand aufzeigen, detektieren, erfolgen. Hierbei sind insbesondere fünf bevorzugte Ausführungsformen zu nennen, die in Zusammenhang mit der vorliegenden Erfindung auch für Hochdurchsatzmethoden (High Trough Put Screening, HTS) geeignet sind:
1. Über Fluoreszenz Korrelations Spektroskopie (FCS) (Proc. Natl. Acad. Sci. USA (1994) 11753-11575) läßt sich die durchschnittliche Diffusionsrate eines Fluoreszenzmoleküls in Abhängigkeit zur Masse in einem kleinen Probenvolumen bestimmen. Durch Messen der Massenänderung bzw. der daraus resultierenden veränderten Diffunsionsrate einer Testverbindung beim Binden an das Enzym läßt sich FCS zur Bestimmung von Protein-Ligand-Wechselwirkungen einsetzten. Ein Testsystem kann direkt zur Messung der Bindung einer durch ein Fluoreszenzmolekül markierten Testverbindung aufgebaut werden. Alternativ kann das Testsystem so konzipiert sein, dass eine durch ein Fluoreszenzmolekül markierte chemische Referenzverbindung durch weitere Testverbindungen verdrängt wird ("Verdrändungsassay"). Die so identifizierten, an das Enzym bindenden Verbindungen sind als Inhibitoren geeignet.
2. Die Fluoreszenzpolarisation nutzt die Eigenschaft eines mit polarisiertem Licht angeregten ruhenden Fluorophors ebenfalls wieder polarisiertes Licht zu emittieren. Kann der Fluorophor allerdings während des angeregten Zustands rotieren, so geht die Polarisation des emittierten Fluoreszenzlichts mehr oder weniger verloren. Bei sonst gleichen Bedingungen (z.B. Temperatur, Viskosität, Lösungsmittel) ist die Rotation eine Funktion der Molekülgröße, womit man über das Messsignal eine Aussage über die Größe des am Fluorophor gebundenen Rests treffen kann (Methods in Enzymology 246 (1995), pp. 283-300). Ein Testsystem kann direkt zur Messung der Bindung einer durch ein Fluoreszenzmolekül markierten Testverbindung an das Enzym aufgebaut werden. Alternativ kann das Testsystem auch in Form des unter 1 beschriebenen "Verdrängungsassays" konzipiert sein. Die so identifizierten, an das Enzym bindenden Verbindungen sind als Inhibitoren geeignet.
3. Fluoreszenz-Resonanz Energie Tranfer" (FRET) basiert auf der strahlungslosen Energieübertragung zwischen zwei räumlich benachbarten Fluoreszenzmolekülen unter geeigneten Bedingungen. Eine Voraussetzung ist die Überlappung des Emissionsspektrums des Donormoleküls mit dem Anregungsspektrum des Akzeptormoleküls. Durch Fluoreszenzmarkierung des Enzyms und den auf Bindung Testverbindungen kann mittels FRET die Bindung gemessen werden (Cytometry 34, 1998, pp. 159-179). Alternativ kann das Testsystem auch in Form des unter 1 beschriebenen "Verdrängungsassays" konzipiert sein. Eine besonders geeignete Ausführungsform der FRET Technologie ist die "Homogenous Time Resolved Fluorescence" (HTRF), wie sie von Packard BioScience vertrieben wird. Die so identifizierten, an das Enzym bindenden Verbindungen sind als Inhibitoren geeignet.
4. Surface Enhanced-Laser Desorption/Ionisation (SELDI) in Kombination mit einem Time of Flight Massenspektrometer (MALDI-TOF) ermöglicht die schnelle Analyse von Molekülen auf einem Träger und kann zur Analyse von Protein-Ligand Wechselwirkungen verwendet werden (Worral et al., (1998) Anal. Biochem. 70:750-756). In einer bevorzugten Ausführungsform immobilisiert man nun das Enzym auf einem geeigneten Träger und inkubiert diesen mit der Testverbindung. Nach einem oder mehreren geeigneten Waschschritten kann die an das Enzym zusätzlich gebundenen Moleküle der Testverbindung mittels der oben erwähnten Methodik detektieren und somit Inhibitoren selektieren. Die so identifizierten, an das Enzym bindenden Verbindungen sind als Inhibitoren geeignet.
5. Die Messung von Oberflächen Plasmonenresonanz basiert auf der Änderung des Brechnungsindexes an einer Oberfläche beim Binden einer Testverbindung an ein auf besagter Oberfläche immobilisierten Protein. Da die Änderung des Brechungsindex für eine definierte Änderung der Massenkonzentration an der Oberfläche quasi für alle Proteine und Polypeptide identisch ist, kann diese Methode prinzipiell auf jedes Protein angewendet werden (Lindberg et al. Sensor Actuators 4 (1983) 299-304; Malmquist Nature 361 (1993) 186-187). Die Messung kann beipielsweise mit Hilfe der von Biacore (Freiburg) vertriebenen auf Oberflächen-Plasmonenresonanz basierenden Analyseautomaten in einem Durchsatz von derzeit bis zu 384 Proben pro Tag durchgeführt werden. Ein Testsystem kann direkt zur Messung der Bindung einer Testverbindung an das erf.-Protein aufgebaut werden. Alternativ kann das Testsystem auch in Form des unter 1 beschriebenen "Verdrängungsassays" konzipiert sein. Die so identifizierten an das Enzym bindenden Verbindungen sind als Inhibitoren geeignet.

Sämtliche, über die oben genannten Verfahren identifizierten Substanzen können anschließend in einer anderen Ausführungsform des erfindungsgemäßen Verfahren auf ihre herbizide Wirkung überprüft werden.

Eine bevorzugte Ausführungsform des Nachweises gemäß Schritt d) ist dadurch gekennzeichnet, dass man die zu testende Verbindung
a) mit einem pflanzlichen Zelllysat, das mindestens eines der Enzyme Tryptophanaminotransferase, Indol-3-pyruvatdecarboxylase und Indol-3-acetaldehydoxidase enthält, oder
b) mit mindestens einem der Enzyme Tryptophanaminotransferase, Indol-3-pyruvatdecarboxylase und Indol-3-acetaldehydoxidase, die entweder partiell oder vollständig gereinigt sind, versetzt und
c) im Anschluß die enzymatische Aktivität von mindestens einem der vorstehend genannten Enzyme im Vergleich zur Aktivität von mindestend einem der vorstehend genannten Enzyme, die nicht mit einer Testverbindung versetzt wurden, ermittelt, wobei die Verbindungen selektiert werden, welche die enzymatische Aktivität von mindestens einem der vorstehend genannten Enzyme reduzieren oder blockieren.

Die in Schritt (b) eingesetzte, partiell oder vollständig gereinigten Enzyme können aus pflanzlichen Zellen nach dem Fachmann bekannten Prozeduren erhalten werden, wie z.B. in Truelsen, T.A. (Physiol. Plant. 28 (1973) 67 - 70) beschrieben. Hierbei können sämtliche Pflanzenspezies, welche die Auxinbiosynthese über den Eingangs beschriebenen Stoffwechselweg realisieren, zur Isolation der Enzyme herangezogen werden. Beispielhaft, aber nicht abschließend seien hier genannt Vertreter aus der Familie der Leguminosae (Fabaceae) wie Mungbohnen (Vigna radiata), Bohne (Phaseolus vulgaris) oder Erbse (Pisum sativum) sowie Vertreter aus der Familie der Gramineae (Poaceae) wie Mais (Zea mays). Weitere Vertreter aus der Familie der Leguminosae sind dem Fachmann bekannt und beispielsweise in Strasburger "Lehrbuch der Botanik", Sitte, P., Ziegler, H., Ehrendorfer, F., Bresinsky, A., Gustav Fischer Verlag, Stuttgart beschrieben.

Zur Identifizierung von Verbindungen in Schritt (c) wird nach einer Reaktionszeit die enzymatische Aktivität des entsprechenden Enzyms im Vergleich zur Aktivität der nicht gehemmten Enyzms ermittelt. Hierbei werden Verbindungen selektiert, die eine signifikante Abnahme der enzymatischen Aktivität zur Folge haben.

Unter Reaktionszeit ist hier die Zeit zu verstehen, die man für die Durchführung eines Aktivitätstests bis zum Erhalt einer signifikanten Aussage über eine Aktivität benötigt. Sie hängt sowohl vonder spezifischen Aktivität des im Test eingesetzten Proteins als auch von der verwendeten Methode und der Empfindlichkeit der verwendeten Geräte ab. Dem Fachmann ist die Ermittlung der Reaktionszeiten bekannt. Bei auf spektroskopischen Methoden basierenden Testsystemen liegen die Reaktionszeiten im allgemeinen zwischen > 0 bis 120 Minuten.

Die Bestimmung der Aktivität kann durch Inkubation des jeweiligen Enzyms mit einem geeigneten Substrat erfolgen, wobei der Umsatz des Substrates oder der Anstieg des entstehenden Produktes spektroskopisch verfolgt wird.

Beispielsweise können Tryptophan, Indol-3-pyruvat und Indol-3-acetaldehyd als Substrate eingesetzt werden. Daneben können auch radioaktive Derivate oder mit chromophoren oder fluorophoren Gruppen modifizierte Derivate des Tryptophan, Indol-3-pyruvat und Indol-3-acetaldehyd in den Verfahren gemäß der vorliegenden Erfindung eingesetzt werden, die zum Beispiel eine spektroskopische Bestimmung ermöglichen.

In Abhängigkeit vom eingesetzten Substrat ergeben sich prinzipiell drei Verfahrensvarianten für die Bestimmung der Enzymatischen Aktivität:

### Verfahrensvariante 1 a)-e):

Verwendet man Tryptophan als Substrat zur Bestimmung zur Bestimmung der enzymatischen Aktivität von mindestens einem der Enzyme ausgewählt aus der Gruppe bestehend aus den Enzymen Tryptophanaminotransferase, Indol-3-pyruvatdecarboxylase und Indol-3-acetaldehydoxidase in Schritt (c) des Verfahrens I, so kann die enzymatische Aktivität über
a) die Abnahme an L-Tryptophan; oder
b) die Zunahme an Indol-3-pyruvat; oder
c) die Zunahme an Indol-3-acetaldehyd; oder
d) die Zunahme an Indol-3-essigsäure; oder
e) eine Kombination von mindestens zwei der Methoden (a) bis (d)
ermittelt werden.

### Verfahrensvariante 2 a)-d):

Verwendet man Indol-3-pyruvat als Substrat zur Bestimmung zur Bestimmung der enzymatischen Aktivität von einem oder beiden Enzymen ausgewählt aus der Gruppe bestehend aus den Enzymen Indol-3-pyruvatdecarboxylase und Indol-3-acetaldehydoxidase in Schritt (c) des Verfahrens I, so kann die enzymatische Aktivität über
a) die Abhahme an Indol-3-pyruvat; oder
b) die Zunahme an Indol-3-acetaldehyd; oder
c) die Zunahme an Indol-3-essigsäure; oder
d) eine Kombination von mindestens zwei der Methoden (a) bis (c) ermittelt werden.

### Verfahrensvariante 3 a)-c):

Verwendet man Indol-3-acetaldehyd als Substrat zur Bestimmung zur Bestimmung der enzymatischen Aktivität der Indol-3-acetaldehydoxidase in Schritt (c) des Verfahrens I, so kann die enzymatische Aktivität über
a) die Abnahme an Indol-3-acetaldehyd; oder
b) die Zunahme an Indol-3-essigsäure; oder
c) eine Kombination der Methoden (a) und (b) ermittelt werden.

Hierbei richtet sich die Wahl der Verfahrensvariante aber auch die in den jeweiligen Verfahrensvarianten erwähnten Methodenkombinationen nach der Zusammensetzung der für den Test eingesetzten Enzymlösung bzw. danach, welche enzymatische Aktivität zu bestimmen ist.

Enthält die für die den Test eingesetzte Enzymlösung die Enzyme Tryptophanaminotransferase, Indol-3-pyruvatdecarboxylase und Indol-3-acetaldehydoxidase so sind folgende Methoden möglich: 1 a), 1 d), 1 e) (e=a+d), 2 a), 2 c), 2 d) (d=a+c) und 3 a), 3 b), 3 c) (c=a+b).

Enthält die für die den Test eingesetzte Enzymlösung Tryptophanaminotransferase und Indol-3-pyruvatdecarboxylase so sind folgende Methoden möglich: 1 a), 1 c), 1 e) (e=a+c), 2 a), 2 b), 2 d) (d=a+b)

Enthält die für die den Test eingesetzte Enzymlösung Indol-3-pyruvatdecarboxylase und Indol-3-acetaldehydoxidase so sind folgende Methoden möglich: 2 a), 2 c), 2 d) (d=a+c) und 3 a), 3 b), 3 c) (c=a+b).

Enthält die für die den Test eingesetzte Enzymlösung Tryptophanaminotransferase so sind folgende Methoden möglich: 1 a), 1 b), 1 e) (e=a+b).

Enthält die für die den Test eingesetzte Enzymlösung Indol-3-pyruvatdecarboxylase so sind folgende Methoden möglich: 2 a), 2 b), 2 d) (d=a+b)

Enthält die für die den Test eingesetzte Enzymlösung Indol-3-acetaldehydoxidase so sind folgende Methoden möglich: 3 a), 3 b), 3 c) (c=a+b).

In einer bevorzugten Ausführungsform der oben genannten Verfahrensvarianten wird zur Bestimmung der enzymatischen Aktivität eine Mischung aus den drei Enzymen Tryptophanaminotransferase, Indol-3-pyruvatdecarboxylase und Indol-3-acetaldehydoxidase verwendet, was wie oben stehend erwähnt folgende Testmethoden ermöglicht: 1 a), 1 d), 1 e) (e=a+d), 2 a), 2 c), 2 d) (d=a+c) und 3 a), 3 b), 3 c) (c=a+b).

Geeignete spektroskopische Methoden für die genannten Verfahren 1, 2 und 3 sind Massenspektroskopie oder UV-VISSpektroskopie, LC/UV-VIS (Flüssigchromatographie/UV-Vis Spektroskopie) oder UV-Vis Spektroskopie). Insbesondere für die jeweiligen Verfahrensvarianten 1b), 1d), 1 e), 2 d) und 3 c) eignet sich Massenspektroskopie wie LC/MS oder HPLC/MS oder diverse auf LC oder HPLC basierende Methoden in Verbindung mit Leitfähigkeitsmessungen, NMR-Messungen oder Messungen des Brechungsindex, da diese die zeitgleiche Bestimmung unterschiedlicher Substanzen ermöglicht.

In einer bevorzugten Ausführungsform erfolgt die photometrische Bestimmung angelehnt an ein von Simpson, R.M.et al. (Planta 201 (1997) 71 - 77) und Truelsen, T.A. et al. (Phsiol. Plant. (1972) 26, 289 - 295) beschriebenes Verfahren, die massenspektroskopische Bestimmung angelehnt an ein von Simpson, R.M. et al.(Planta 201 (1997), 71 - 77) beschriebenes Verfahren.

In einer besonders bevorzugten Ausführungsform wird die Zu- bzw. Abnahme von Tryptophan und/oder Indol-3-pyruvat und/oder die Zunahme von Indol-3-essigsäure und/oder Indol-3-buttersäure zur Bestimmung der enzymatischen Aktivität photometrisch ermittelt.

Die photometrischen Bestimmung kann beispielsweise durch die Zu- bzw. Abnahme an Indol-3-pyruvat bei der Wellenlänge λ= 328nm detektiert werden (Simpson, R.M., Nonhebel, H.M., Christie, D.L. (1997), Planta 201, 71 - 77 , Truelsen,T.A. (1972), Phsiol. Plant., 26, 289 - 295).

Die Zu- oder Abnahme an Indol-3-essigsäure kann mittels Fluoreszenz detektiert werden (Ex = 254nm; Em = 360nm; nach Mazur H. et al, J. Appl. Phycology 13 (2001) 35-42).

Zudem können sämtliche, in den vorstehend genannten Verfahren identifizierten Inhibitoren der Enzyme in einem in vivo Aktivitätstest auf ihre herbizide Wirkung überprüft werden. Hierbei wird die entsprechende Substanz zur Überprüfung der herbiziden Wirkung auf die entsprechende Schadpflanze appliziert.

Alle oben beschriebenen Verfahren werden im folgenden mit dem Begriff "erfindungsgemäßes Verfahren" beschrieben.

Das erfindungsgemäße Verfahren kann in einzelnen getrennten Verfahrensansätzen und/oder vorteilhaft gemeinsam oder besonders vorteilhaft in einem High-Throughput-Screening durchgeführt werden und zur Identifizierung von Verbindungen mit herbizider Wirkung verwendet werden.

In dem erfindungsgemäßen Verfahren können auch mehrere Testverbindungen eingesetzt werden. Wenn durch eine Gruppe von Testverbindungen eine Beeinflussung des Targets erfolgt, dann ist es entweder möglich, die einzelnen Testverbindungen direkt zu isolieren oder die Gruppe von Testverbindungen in verschiedene Untergruppen teilen, z.B. wenn sie aus einer Vielzahl von verschiedenen Komponenten besteht, um so die Zahl der verschiedenen Testverbindungen im Testsystem zu reduzieren. Das erfindungsgemäße Verfahren wiederholt man dann mit der einzelnen Testverbindung oder der entsprechenden Untergruppe von Testverbindungen. Abhängig von der Komplexität der Probe können die oben beschriebenen Schritte mehrmals wiederholt werden, vorzugsweise bis die gemäß der erfindungsgemäßen Methode identifizierte Untergruppe nur noch eine geringe Anzahl von Testverbindungen oder nur noch eine Testverbindung umfaßt.

Weiterer Gegenstand der Erfindung sind Verbindungen identifiziert nach den erfindungsgemäßen Verfahren. Diese Verbindungen werden im folgenden "selektierte Verbindungen" genannt. Sie weisen ein Molekulargewicht von kleiner 1000 g/mol, vorteilhaft kleiner 900 g/mol, bevorzugt kleiner 800 g/mol, besonders bevorzugt kleiner 700 g/mol, ganz besonders bevorzugt kleiner 600 g/mol und einem Ki-Wert kleiner 1 mM auf.

Die über die erfindungsgemäßen Verfahren identifizierten Verbindungen können natürlich auch in Form ihrer landwirtschaft brauchbaren Salze vorliegen.

Unter landwirtschaftlich brauchbaren Salzen kommen vor allem die Salze derjenigen Kationen oder die Säureadditionssalze derjenigen Säuren in Betracht, deren Kationen beziehungsweise Anionen die herbizide Wirkung der über die erfindungsgemäßen Verfahren identifizierten Verbindungen mit herbizider Wirkung nicht negativ beeinträchtigen.

Ferner können die selektierte Verbindungen sofern sie asymmetrisch substituierte α-Kohlenstoffatome enthalten, entweder als Racemate, Enantiomerengemische, reine Enantiomere oder, sofern sie chirale Substituenten aufweisen, auch als Diastereomerengemische vorliegen.

Die selektierten Verbindungen können chemisch synthetisierte oder mikrobiologisch produzierte Stoffe sein und z.B. in Zellextrakten von z.B. Pflanzen, Tieren oder Mikroorganismen auftreten. Das Reaktionsgemisch kann ein zellfreier Extrakt sein oder eine Zelle oder Zellkultur umfassen. Geeignete Methoden sind dem Fachmann bekannt und werden z.B. allgemein beschrieben in Alberts, Molecular Biology the cell, 3rd Edition (1994), z.B. Kapitel 17.

Mögliche Testverbindungen können Expressionsbibliotheken wie z.B. cDNA-Expressionsbibliotheken, Peptide, Proteine, Nukleinsäuren, Antikörper, kleine organische Stoffe, Hormone, PNAs oder ähnliches sein (Milner, Nature Medicin 1 (1995), 879-880; Hupp, Cell. 83 (1995), 237-245; Gibbs, Cell. 79 (1994), 193-198 und darin zitierte Referenzen).

Die selektierte Verbindungen können zur Bekämpfung von unerwünschtem Pflanzenwuchs sowie unter Umständen auch zur Defoliation, beispielsweise von Kartoffeln, oder Desikkation, beispielsweise von Baumwolle verwendet werden. Des weiteren können die selektierten Verbindungen ggf. auch zur Regualtion des Wachstums von Pflanzen verwendet werden, da Inhibitoren der Biosynthese des Pflanzenhormons Auxins einen Einfluß auf das Wachstum der Pflanzen haben können. Agrochemische Zusammensetzungen, welche die selektierten Verbindungen enthalten, bekämpfen Pflanzenwuchs auf Nichtkulturflächen sehr gut, besonders bei hohen Aufwandmengen. In Kulturen wie Weizen, Reis, Mais, Soja und Baumwolle wirken sie gegen Unkräuter und Schadgräser, ohne die Kulturpflanzen nennenswert zu schädigen. Dieser Effekt tritt vor allem bei niedrigen Aufwandmengen auf. Die selektierten Verbindungen können zur Bekämpfung der oben bereits erwähnten Schadpflanzen verwendet werden.

In Abhängigkeit von der jeweiligen Applikationsmethode können selektierten Verbindungen bzw. diese enthaltende agrochemische Zusammensetzungen vorteilhaft noch in einer weiteren Zahl von Kulturpflanzen zur Beseitigung unerwünschter Pflanzen eingesetzt werden. In Betracht kommen beispielsweise folgende Kulturen:

Allium cepa, Ananas comosus, Arachis hypogaea, Asparagus officinalis, Beta vulgaris spec. altissima, Beta vulgaris spec. rapa, Brassica napus var. napus, Brassica napus var. napobrassica, Brassica rapa var. silvestris, Camellia sinensis, Carthamus tinctorius, Carya illinoinensis, Citrus limon, Citrus sinensis, Coffea arabica (Coffea canephora, Coffea liberica), Cucumis sativus, Cynodon dactylon, Daucus carota, Elaeis guineensis, Fragaria vesca, Glycine max, Gossypium hirsutum, (Gossypium arboreum, Gossypium herbaceum, Gossypium vitifolium), Helianthus annuus, Hevea brasiliensis, Hordeum vulgare, Humulus lupulus, Ipomoea batatas, Juglans regia, Lens culinaris, Linum usitatissimum, Lycopersicon lycopersicum, Malus spec., Manihot esculenta, Medicago sativa, Musa spec., Nicotiana tabacum (N.rustica), Olea europaea, Oryza sativa, Phaseolus lunatus, Phaseolus vulgaris, Picea abies, Pinus spec., Pisum sativum, Prunus avium, Prunus persica, Pyrus communis, Ribes sylestre, Ricinus communis, Saccharum officinarum, Secale cereale, Solanum tuberosum, Sorghum bicolor (s. vulgare), Theobroma cacao, Trifolium pratense, Triticum aestivum, Triticum durum, Vicia faba, Vitis vinifera, Zea mays.

Darüber hinaus können die selektierten Verbindungen auch in Kulturen, die durch Züchtung einschließlich gentechnischer Methoden gegen die Wirkung von Herbiziden tolerant sind, verwandt werden.

Weiter Gegenstand der Erfindung ist ein Verfahren zur Herstellung der oben bereits erwähnten agrochemischen Zusammensetzung, dadurch gekennzeichnet dass man selektierten Verbindungen mit für die Formulierung geeigneten Hilfsmitteln formuliert.

Die selektierten Verbindungen können z.B. in Form von direkt versprühbaren wässrigen Lösungen, Pulvern, Suspensionen, auch hochprozentigen wäßrigen, öligen oder sonstigen Suspensionen bzw. Suspoemulsionen oder Dispersionen, emulgierbaren Konzentraten, Emulsionen, Öldispersionen, Pasten, Stäubemitteln, Streumitteln oder Granulaten formuliert werden und durch Versprühen, Vernebeln, Verstäuben, Verstreuen oder Gießen angewendet werden. Die Anwendungsformen richten sich nach den Verwendungszwecken und der Natur der selektierten Verbindungen und sollten in jedem Fall möglichst die feinste Verteilung der selektierten Verbindungen gewährleisten. Die agrochemischen Zusammensetzung enthalten eine herbizid wirksame Menge mindestens einer selektierten Verbindung und für die Formulierung von agrochemischen Zusammensetzungen übliche Hilfsstoffe.

Zur Herstellung von Emulsionen, Pasten oder wässrigen oder ölhaltigen Formulierungen sowie dispergierbaren Konzentraten (DC) können die selektierten Verbindungen in einem einem Öl oder Lösungsmittel gelöst oder dispergiert werden, wobei weitere Formulierungshilfsstoffe zur Homogenisierung zugesetzt werden können. Es können aber auch aus selektierter Verbindung, gegebenenfalls Lösungsmitteln oder Öl sowie optional weiteren Hilfsmitteln bestehende flüssige oder feste Konzentrate hergestellt werden, die zur Verdünnung mit Wasser geeignet sind. Hier zu nennen sind emulgierbare Konzentrate (EC, EW), Suspensionen (SC), lösliche Konzentrate (SL), dispergierbaren Konzentrate (DC), Pasten, Pastillen netzbaren Pulvern oder Granulaten, wobei die festen Formulierungen entweder in Wasser löslich (soluble) oder dispergierbar (wettable) sein können. Desweiteren können entsprechende Pulver bzw. Granulate oder Tabletten noch mit einem festen, den Abrieb oder eine vorzeitige Wirkstofffreisetzung verhinderenden Überzug ("coating") versehen werden.

Prinzipiell sind unter dem Begriff Hilfsmittel folgende Substanzklassen zu verstehen: Antischäumungsmittel, Verdicker, Netz-, Haftmittel, Dispergiermittel, Emulgiermittel, Bakterizide und/oder thixotrophe Agentien. Die Bedeutung der oben genannten Mittel ist dem Fachmann bekannt.

SLs, EWs und ECs können durch einfaches Mischen der entsprechenden Inhaltsstoffe hergestellt werden, Pulver über Mischen oder Vermahlen in speziellen Mühlentypen (z.Bsp. Hammermühlen). DC, SCs und SEs werden üblicherweise über Naßvermahlung ("wet milling") hergestellt, wobei ein SE aus einem SC durch Zugabe einer organischen Phase, die weitere Hilfsmittel oder selektierte Verbindungen enthalten kann, hergestellt werden kann. Die Herstellung ist bekannt. Pulver-, Streu- und Stäubemittel können vorteilhaft durch Mischen oder gemeinsames Vermahlen der wirksamen Substanzen mit einem festen Trägerstoff hergestellt werden. Granulate, z.B. Umhüllungs-, Imprägnierungs- und Homogengranulate können durch Bindung der selektierten Verbindungen an feste Trägerstoffe hergestellt werden. Weitere Details der Herstellung sind dem Fachmann bekannt, und z.Bsp. in folgenden Schriften aufgeführt: US 3,060,084, EP-A 707445 (für flüssige Konzentrate), Browning, "Agglomeration", Chemical Engineering, Dec. 4, 1967, 147-48, Perry's Chemical Engineer's Handbook, 4th Ed., McGraw-Hill, New York, 1963, pages 8-57 und ff. WO 91/13546, US 4,172,714, US 4,144,050, US 3,920,442, US 5,180,587, US 5,232,701, US 5,208,030, GB 2,095,558, US 3,299,566, Klingman, Weed Control as a Science, John Wiley and Sons, Inc., New York, 1961, Hance et al., Weed Control Handbook, 8th Ed., Blackwell Scientific Publications, Oxford, 1989 und Mollet, H., Grubemann, A., Formulation technology, Wiley VCH Verlag GmbH, Weinheim (Federal Republic of Germany), 2001.

Inerte flüssige und/oder feste Trägerstoffe geeignet für die erfindungsgemäßen Formulierungen sind dem Fachman in einer Vielzahl bekannt, wie z.Bsp. flüssige Zusatzstoffe wie Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Paraffin, Tetrahydronaphthalin, alkylierte Naphthaline oder deren Derivate, alkylierte Benzole oder deren Derivate, Alkohole wie Methanol, Ethanol, Propanol, Butanol, Cyclohexanol, Ketone wie Cyclohexanon oder stark polare Lösungsmittel, z.B. Amine wie N-Methylpyrrolidon oder Wasser.

Feste Trägerstoffe sind beispielsweise Mineralerden wie Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte wie Getreidemehl, Baumrinden-, Holz- und Nußschalenmehl, Cellulosepulver oder andere feste Trägerstoffe.

Oberflächenaktive Stoffe (Tenside) geeignet für die erfindungsgemäßen Formulierungen sind dem Fachman in einer Vielzahl bekannt, wie z.Bsp. Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenyl-, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether oder Polyoxypropylenalkylether, Laurylalkoholpolyglykoletheracetat, Sorbitester, Lignin-Sulfitablaugen oder Methylcellulose.

Die Applikation der agrochemischen Zusammensetzungen bzw. der selektierten Verbindungen kann im Vorauflauf- oder im Nachauflaufverfahren erfolgen. Sind die selektierten Verbindungen für gewisse Kulturpflanzen weniger verträglich, so können Ausbringungstechniken angewandt werden, bei welchen die selektierten Verbindungen mit Hilfe der Spritzgeräte so gespritzt werden, daß die Blätter der empfindlichen Kulturpflanzen nach Möglichkeit nicht getroffen werden, während die selektierten Verbindungen auf die Blätter darunter wachsender unerwünschter Pflanzen oder die unbedeckte Bodenfläche gelangen (post-directed, lay-by).

Die Aufwandmengen an selektierten Verbindungen betragen je nach Bekämpfungsziel, Jahreszeit, Zielpflanzen und Wachstumsstadium 0.001 bis 3.0, vorzugsweise 0.01 bis 1.0 kg/ha.

Die Erfindung wird durch die nachstehenden Beispiele weiter veranschaulicht, die nicht als beschränkend aufgefaßt werden sollten.

Beispiel 1 - Enzymisolierung der Tryptophanaminotransferase (TA-Tase) aus etiolierten Mungbohnen (nach Simpson, R.M., Nonhebel, H.M., Christie, D.L. (1997), Planta 201, 71 - 77)

Die Mungbohnen ( Vigna radiata ) werden für 6-7 Tage in Vermiculite im Dunkeln angezogen. Die Pflanzen sind zum Erntezeitpunkt ca. 8 cm hoch.

Sämtliche folgenden Arbeitsschritte finden bei 4°C statt. 100g frisch geerntetes Pflanzenmaterial wird bei 4°C mit 100 ml Extraktionspuffer (50mM KH₂PO₄, pH 8.5; 0.5mM EDTA; 0.5mM MnCl₂; 10mM Isoascorbat) in 2 Schritten von je 1 min mit einem Mixer homogenisiert. Danach wird 5 g Polyvinylpolypyrolidon zugesetzt (Verhätnis Pflanzenmat./ PVPP 100 : 5 ), 5 min gerührt, anschließend über 8 lagige Gaze filtriert. Nach Zentrifugation des Filtrates (20000g, 4°C, 20min; SL-250 T Rotor - Sorvall Super T 1 Zentrifuge) wird der Überstand einer fraktionierten Ammoniumsulfatfällung (60%, 80%). Der verbleibende Überstand wird verworfen, das Pellet mit 3ml Säulenpuffer (10mM Tris-HCl, pH 8,0; 0,1M NaCl) resuspendiert und auf ein Gesamtvolumen von 5,0 ml gebracht und über eine äquilibrierte PD 10 Säule (Ammersham Pharmacia) entsalzt. Die entsalzte Enzymlösung wird je nach Verwendung portionsweise bei -20°C eingefroren, kann aber je nach Bedarf auch direkt in den Enzymassay eingesetzt werden.

### Beispiel 2 - Enzymassay

In einem geigneten Gefäß werden 500µl Assaypuffer (0.1M Borax, pH 8,5; 10mM Tryptophan, 0.5mM Na-Arsenat, 0.5mM EDTA, 50µM Pyrodoxalphosphat), 100µl einer frisch angesetzten 10mM α-Ketoglutaratlösung und ca. 50-250µl Enzymlösung (5mg/ml) eingesetzt. Man ergänzt den Assay mit Wasser sowie 10µl Wirkstofflösung ad 1 ml, wobei die zugegebene Menge an Wirkstofflösung zwischen 1-5% des Gesamtansatzvolumens liegen und 1mM nicht überschreiten sollte, und inkubiert 30 Minuten unter Schütteln bei 60°C. Anschließend wird die Reaktion mit 150µl Methanol gestoppt und die Probe bei 20000g / 5 Minuten zentrifugiert. Der Überstand wird mittels LCMS
- Methode analysiert, wobei die folgenden Geräte/Parameter verwendet werden (hier sollte doch die Angabe des Gerätetypes alle weiteren Parameter überflüssig machen):
   - Geräte:: Pumpen Bio-Tek 522 und 520
   Säulenthermostat Bio-Tek 582
   Autosampler Bio-Tek 560
   UV-Detector Bio-Tek 535
   Massendetektor Applied Biosystems Mariner
   - Säule:: Phenomenex Aqua 3µ C18 125 A, 150 x 2 µm
   Flußrate: 0,3 ml/min
   Laufmittel:
   Puffer A: 0,2% Essigsäure
   Puffer B: Methanol
   - verwendeter Gradient:: 0 - 30 min 75% auf 50% linear Puffer A
   30 - 35 min 50% Puffer A
   35 - 40 min 50% auf 75% linear Puffer A
   40 - 45 min 75 % Puffer A
   - UV-Detektor:: 280 nm

Die für die Auswertung benötigten Retentionszeiten sind in Tabelle 1 zusammengefaßt:

**Tabelle 1**

| Verbindung | Retentionszeiten: |
|---|---|
| L-Tryptophan | 3,8 min |
| Indol-3-essigsäure | 23,7 min |
| Indol-3-pyruvat | 35,2 min 42,5 min *) |

| | |
|---|---|
| *) hierbei handelt es sich um Ketoenoltautomerie | |

### Beispiel 3 - Herbizide Wirkung von Verbindung I

Die herbizide Wirkung der Verbindung I ließ sich durch Gewächshausversuche zeigen:

Als Kulturgefäße dienten Plastiktöpfe mit lehmigem Sand mit etwa 3,0% Humus als Substrat. Die Samen der Testpflanzen wurden nach Arten getrennt eingesät.

Bei Vorauflaufbehandlung wurden die in Wasser suspendierten oder emulgierten Wirkstoffe direkt nach Einsaat mittels fein verteilender Düsen aufgebracht. Die Gefäße wurden leicht beregnet, um Keimung und Wachstum zu fördern, und anschließend mit durchsichtigen Plastikhauben abgedeckt, bis die Pflanzen angewachsen waren. Diese Abdeckung bewirkt ein gleichmäßiges Keimen der Testpflanzen, sofern dies nicht durch die Wirkstoffe beeinträchtigt wurde.

Zum Zweck der Nachauflaufbehandlung wurden die Testpflanzen je nach Wuchsform erst bis zu einer Wuchshöhe von 3 bis 15 cm angezogen und dann mit den in Wasser suspendierten oder emulgierten Wirkstoffen behandelt. Die Testpflanzen wurden dafür entweder direkt gesät und in den gleichen Gefäßen aufgezogen oder sie wurden erst als Keimpflanzen getrennt angezogen und einige Tage vor der Behandlung in die Versuchsgefäße verpflanzt. Die Aufwandmenge für die Nachauflaufbehandlung betrug 3 kg a.S./ha.

Die Pflanzen wurden artenspezifisch bei Temperaturen von 10 - 25°C bzw. 20 - 35°C gehalten. Die Versuchsperiode erstreckte sich über 2 bis 4 Wochen. Während dieser Zeit wurden die Pflanzen gepflegt, und ihre Reaktion auf die einzelnen Behandlungen wurde ausgewertet.

Bewertet wurde nach einer Skala von 0 bis 100. Dabei bedeutet 100 kein Aufgang der Pflanzen bzw. völlige Zerstörung zumindest der oberirdischen Teile und 0 keine Schädigung oder normaler Wachstumsverlauf.

Die in den Gewächshausversuchen verwendeten Pflanzen setzten sich aus folgenden Arten zusammen:

| Lateinischer Name | Deutscher Name | Englischer Name |
|---|---|---|
| Chenopodium album | Weißer Gänsefuß | common lambsquarters |
| Echinocloa crus-galli | Hühnerhirste | cockspur(grass) |
| Pharbitis purpurea | Purpurtrichterwinde | Moringglory, common |

Die Verbindung I zeigte im Nachauflauf eine sehr gute herbizide Wirksamkeit gegen Chenopodium album, Echinocloa crus-galli und Pharbitis purpurea bei Aufwandmengen von 1.0 kg/ha a.S.

### Beispiel 4 - Enzymassay zur Suche nach Hemmstoffen der Indol-3-essigsäure (IES, Auxin) Biosynthese

Zur Gewinnung des Enzymextraktes, wurden 2g eingefrorene, mit flüssigem Stickstoff gemörserte Pflanzensprosse von Chenopodium album (Weißer Gänsefuß) in 3ml 100mM EPPS-Extraktionspuffer (5mM Dithiothreitol, 6µM Pyridoxalphosphat, 10µM Leupeptin, 10µM Pefabloc SC, pH 8,5) zusammen mit einer Spatelspitze Polyvinylpyrolidon über 30min unter leichtem Rühren bei Raumtemperatur aufgetaut. Danach wurde das Pflanzenmaterial für 10min bei 4°C zentrifugiert und der Überstand auf einer Sephadex G-25 Säule (voräquilibriert mit 5mM EPPS-Eluationspuffer (1mM Dithiothreitol, 6µM Pyridoxalphosphat, 10µM Pefabloc SC, pH 8,5) entsalzt. Der erhaltene Enzymextrakt (400µl) wurde unter Zugabe von 100µl EPPS-Extraktionspuffer und 6µl Wirkstofflösung (10mM Verbindung I in DMSO) im Testassay (600µl Volumen) in Gegenwart von 20µM Pyridoxalphosphat, 80mM EPPS-Extraktionspuffer, 50µM L-Tryptophan, 50µM α-Ketoglutarat und 50µM Indol für 2h bei 37°C inkubiert. Der Kontrolle ohne Verbindung I wurde die entsprechende Menge DMSO zugesetzt. Anschließend wurde die Reaktion durch Zugabe von 20µl 7,2N HCl und 3ml Ethylacetat abgestoppt. Die Kontroll-assays ohne Inkubation wurden sofort nach Ansatz auf Eis mit HCl und Ethylacetat behandelt. Nach nochmaliger Extraktion mit 3ml Ethylacetat werden die organischen Phasen (6ml) vereinigt, unter Stickstoffstrom aufkonzentriert und die Proben mit Diazomethan methyliert. Dadurch wurde die im Probenmaterial vorhandene Indol-3-essigsäure in Indol-3-essigsäure-Methylester (IES-Me) überführt. IES-Me wurde anschließend immunoanalytisch durch monoklonale Antikörper (100% reaktiv gegen IES-Me) quantifiziert (nach Weiler E.W., Eberle J., Mertens R., Atzorn R., Feyerabend M., Jourdan P.S., Arnscheidt A., Wieczorek U., in: Immunology in Plant Science (Wang T.L., Ed.), Society for Experimental Biology, Seminar Series 29, Cambridge University Press, Cambridge, 1986, pp. 27-58) und als Maß für die Enzymaktivitäten bestimmt. Die Hemmung der Indol-3-essigsäure (IES)-Synthesereaktion durch Verbindung 1 ist in Tabelle 2 dargestellt.

**Tabelle 2**

| | pMol IES-Me/g Sprossfrischmasse² | Hemmung [%]¹ |
|---|---|---|
| Kontrolle ohne Inkubation | 48,7 +/- 4,5 | |
| Kontrolle mit Inkubation | 445,3 +/- 33,9 | 0 |
| Assay mit Inkubation und 100µM Verbindung I | 156,0 +/- 14,8 | 73 |

| | | |
|---|---|---|
| ¹ Zur Berechnung der % Hemmung wird von den Werten der Kontrollwert ohne Inkubation subtrahiert. ² Mittelwert aus drei Messungen | | |

## Patentansprüche

1. Verfahren zur Herstellung einer agrochemischen Zusammensetzung, **dadurch gekennzeichnet dass** man
i. einen Wirkstoff über ein Verfahren, umfassend die folgenden Schritte:
a) Inkontaktbringen von einem oder mehreren Enzymen ausgewählt aus der Gruppe bestehend aus den Enzymen Tryptophanaminotransferase, Indol-3-pyruvatdecarboxylase und Indol-3-acetaldehydoxidase mit einer oder mehreren Testsubstanzen unter Bedingungen, die die Bindung der Testsubstanz(en) an eines der vorstehend genannten Enzyme oder an die Nukleinsäuresequenz, die eines vorstehend genannten Enzyme kodiert, erlauben; und
b) Nachweis, ob die Testsubstanzen die Transkription, Translation oder Expression von mindestens einem der vorstehend genannten Enzyme reduzieren oder blockieren; oder
c) Nachweis, ob die Testsubstanzen die Aktivität von mindestens einem der vorstehend genannten Enzyme reduzieren oder blockieren; oder
d) Nachweis, ob die Testsubstanz an eines der vorstehend genannten Enzyme bindet
identifiziert und
ii. diesen Wirkstoff mit für die Formulierung von agrochemischen Zusammensetzungen geeigneten Hilfsmitteln formuliert.

2. Verfahren zur Bekämpfung von unerwünschtem Pflanzenwuchs und/oder zur Regulation des Wachstums von Pflanzen, **dadurch gekennzeichnet, daß** man eine agrochemische Zusammensetzung erhältlich über das in Anspruch 1 genannte Verfahren auf Pflanzen, deren Lebensraum und/oder auf Samen einwirken läßt.

3. Verwendung einer agrochemischen Formulierung erhältlich über das in Anspruch 1 genannte Verfahren zur Bekämpfung von unerwünschtem Pflanzenwuchs und/oder zur Regulation des Wachstums von Pflanzen gemäß Anspruch 2.
